# EUROPEAN PATENT APPLICATION

(11) **EP 0 573 158 A1**
(43) Date of publication of application: **08.12.1993**
(21) Application number: 93303690.7
(22) Date of filing: 12.05.1993
(51) Int. Cl.: A61B 1/00, A61B 1/04, A61B 1/06

(54) **Endoscope with internal light source**

(30) Priority: 01.06.1992 US 891888
(71) Applicant: CITATION MEDICAL CORPORATION, Reno, Nevada 89502 (US)
(72) Inventor: Clayton, John B., Reno, Nevada 89509 (US); Bornhop, Daryl J., Truckee, California 96161 (US); Middle, George H., Reno, Nevada 89509 (US)
(74) Representative: Coxon, Philip

(57) **Abstract**

A hand-held endoscope having an internal light source (35) includes a housing (30) and a scope assembly (32) attached to the housing which includes a disposable scope (38), a fiber optic image guide (58) and a plurality of optical illumination fibers formed in a fiber bundle (48). The scope is insertable into a body for illuminating and imaging the internal structure of the body. The illumination fiber bundle is in light communication with an internal light source mounted within the housing. The light source preferably includes a light bulb (36) and a light transmission means (39) that is adapted to direct light from the light bulb into the illumination fiber bundle. The endoscope is constructed to utilize essentially all of the light produced by the light bulb and to remove heat generated by the light bulb.

## Description

### TECHNICAL FIELD

The present invention relates generally to medical devices and more particularly to endoscopes. The present invention is particularly although not exclusively related to an improved endoscope construction such as for an arthroscope in which a light source for the endoscope assembly is internally mounted within a hand-held housing of the arthroscope.

### BACKGROUND OF THE INVENTION

Modern medical techniques which are relatively non-invasive have been recently developed for viewing and performing different medical procedures on the interior structure of body parts. Medical instruments for performing these procedures are known generally as endoscopes and specifically include arthroscopes, spinalscopes, laproscopes, esophagoscopes and others. These instruments typically include a scope which is inserted into the body part to be examined. With an arthroscope, for example, the scope is coupled to a camera assembly and the camera assembly, in turn, is connected to a video display for generating a picture of the interior structure of the joint. Consequently, the operator of the arthroscope is able to view, in real-time, the interior structure of the body as the scope is inserted into the body. By viewing the internal body structure, a diagnosis can be made and appropriate treatment prescribed.

For illuminating the interior body structure, the scope of an endoscope instrument or endoscope assembly of an instrument includes optical illumination fibers that terminate at the distal or viewing end of the scope and direct light into the body. The optical illumination fibers in turn, are coupled to a high power light source that includes a light bulb (i.e. xenon or metal halide). Such a light source, because of its size, power and control requirements is typically mounted externally to the probe and hand-held components of the endoscope. With an arthroscope, for instance, the scope of the endoscope and a portion of the camera assembly are mounted in a hand held housing, and the light source is external to this housing in a separate console.

Such an arrangement necessitates a flexible light transmissive conduit which connects the external light source to the illumination fibers within the scope assembly. These types of light transmissive conduits must be precisely connected to the scope assembly and to the external light source. This complicates the construction of the arthroscope assembly. In addition, such a flexible conduit can detract from the portability of the arthroscope and can limit the range of motion of the scope and hand held housing of the arthroscope.

The present invention seeks to overcome the typical problems associated with an external light source by providing a scope assembly that includes an internal light source. Such a scope assembly allows a medical instrument such as an arthroscope (or other types of endoscopes) to be constructed with a light source that is mounted within the hand held housing of the arthroscope. This eliminates the bulky and complicated external light source and the light transmissive conduits to the external light source.

In addition, with an endoscope assembly having an internal light source, medical instruments, such as arthroscopes can be constructed with improved portability and with far less power consumption. In particular the endoscope can be more freely moved without an external light source and can include a battery pack as an internal power source.

Moreover an instrument such as an arthroscope can include an internal light source as well as a power supply and a microscopic eyepiece. As an example, a microscopic eyepiece can be used to focus on a lens assembly of the endoscope for portable viewing of an image. Such an endoscope can be totally self contained without any external connections.

Accordingly it is an object of the present invention to provide an endoscope assembly which includes an internal light source. It is another object of the present invention to provide an endoscope assembly in which an external light source and light transmissive conduits for connecting the endoscope assembly to such an external light source are eliminated. It is a further object of the present invention to provide an endoscope assembly with an internal light source in which an image produced by the endoscope is substantially equivalent to that of an endoscope having an external high power light source. It is yet another object of the present invention to provide an endoscope assembly constructed to optimize the transmission of light from a miniature internal light source. Finally, it is an object of the present invention to provide an endoscope assembly with an internal light source constructed to dissipate heat generated by the internal light source

### SUMMARY OF THE INVENTION

In accordance with the present invention an improved endoscope assembly having an internal light source is provided. In an illustrative embodiment of the invention, the endoscope is constructed as an arthroscope for examining the joints of the body. Alternately the inventive concepts disclosed herein may be used in connection with other types of endoscopes and medical instruments for viewing other body structures (i.e. spinalscopes, laproscopes, esophagoscopes).

The improved endoscope assembly, generally stated, includes, a hand held housing having an internal light source, an arthroscope scope assembly coupled to the light source, and a camera assembly. The internal light source includes a micro light and light transmitting means for efficiently transmitting light from the micro light to light illumination fibers mounted within the arthroscope scope assembly. The light transmitting means may include an integrating sphere, a parabolic reflecting mirror, a tapered light conduit, and an illumination GRIN lens. This construction utilizes essentially all of the light generated by the micro light and eliminates a separate external light source and a light conduit from the housing to the light source. Heat elimination means is also provided for eliminating heat generated by the micro light within the housing.

The scope assembly of the arthroscope provides a conduit for light for illuminating the internal body structure and a return conduit for images from the internal body structure. The scope assembly is disposable and detachably mounted to the housing and may be malleable, flexible or semi-flexible in construction depending on the application. The scope assembly includes a cylindrically shaped GRIN lens attached to an image guide. The image guide collects the image and transfers this image from a front plane (i.e. image at the GRIN lens of the internal body structure) to another plane depending upon the length of the image guide. The image guide is optically coupled to focusing optics which in turn are optically coupled to the camera assembly. An externally mounted camera control unit controls operation of the camera assembly. In addition, the camera control unit can control a CRT or other visual display device to display an image of the internal structure of the joint.

An arthroscope constructed in accordance with the invention is portable and can be hand held by the physician or other medical personnel. In several alternate embodiments of the invention arthroscopes can be constructed to be totally portable and may include an internal power source. Moreover arthroscopes may be constructed with an internal source, an internal power source, and a microscopic eye piece and be totally self contained.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts.
Figure 1 is a perspective view of a portable endoscope constructed in accordance with the present invention with an internal light source and showing a prior art external light source in phantom;
Figure 2 is a partial cross-sectional view taken along section line 2-2 of Figure 1;
Figure 2A is an enlarged portion of Figure 2 of a front tube component of the housing for mounting the light source;
Figure 3A-3D are schematic views illustrating various light sources for an endoscope constructed in accordance with the present invention;
Figure 4A-4D are schematic views illustrating various heat dissipation means for the arthroscope of Figure 1;
Figure 5A-5C are schematic views illustrating several alternate embodiments of arthroscopes constructed in accordance with the present invention; and
Figure 5D is a prior art arthroscope having an external light source showing a light transmittive connection to the external light source.

### DESCRIPTION OF PREFERRED EMBODIMENT

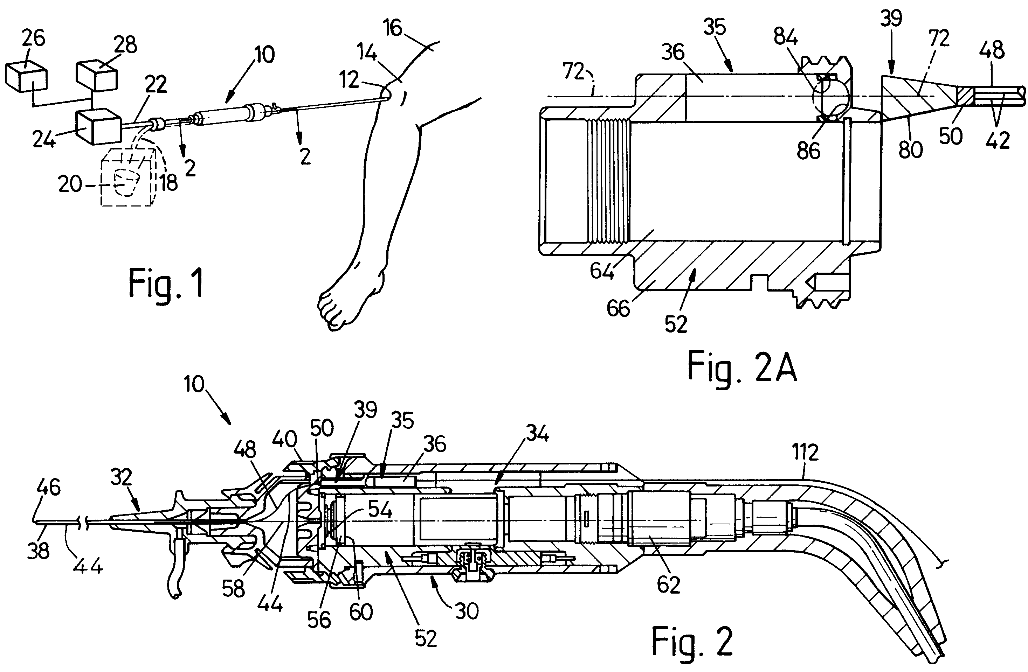

With reference to Figure 1, an arthroscope 10 is adapted to be inserted into an entry site 12 such as into a knee 14 of a patient 16 to examine the internal structure of the knee 14. A prior art arthroscope would be in light communication via optical conduit 18 to an external light source 20 that may include a high powered quartz, halogen, or metal halide lamp. These optical conduit 18 and light source 20 components (18,20) are shown in dashed lines because they are replaced by an internal light source of the invention.

Figure 1 also shows that the arthroscope 10 is electrically connected via electrical line 22 to a camera control unit 24. Camera control unit 24, is in turn, electrically connected to a cathode ray tube (CRT) 26 and to a video camera recorder (VCR) 28, for respectively displaying and recording a video image of the internal structure of the knee 14.

Referring now to Figure 2, the improved arthroscope 10 is shown in cross section and includes a housing 30, a disposable arthroscope scope assembly 32 detachably mounted to the housing 30, and a camera assembly 34 mounted within the housing 30. An internal light source 35 including a micro light 36 or miniature light bulb is also mounted within the housing 30 in light communication with the arthroscope scope assembly 32. The internal light source 35 also includes light transmitting means 39 for transmitting light from the micro light 36 to the arthroscope scope assembly 32. The light transmitting means 39 is adapted to maximize transmission of the light from the micro light 36 to the scope assembly 32 and may include a number or all of the components to be hereinafter described.

The construction of the scope assembly 32 and camera assembly 34 are essentially the same as the arthroscope described in more detail in application serial number 07/651,746 entitled "Portable Arthroscope With Disposable Probe", assigned to the assignee of the present application and included herein by reference.

Briefly stated, the arthroscope scope assembly 32 includes a scope 38 that is adapted for insertion at the entry site 12 of a joint such as the knee 12 (Figure 1). The scope 38 may be constructed as a malleable, flexible or semi-flexible member. As such it may include a metal cannula, or a flexible polyimide tube as an outer member. In addition, the arthroscope scope assembly 32 is disposable and may be removably attached to the housing 30 using a threaded ring clamp 40. Such a threaded ring clamp 40 is more particularly described in the above cited patent application. In general such a ring clamp 40 provides a removable attachment or attaching the arthroscope scope assembly with the optical components in a proper alignment.

The arthroscope scope assembly 32 also includes a plurality of illumination fibers 42 (Figure 2A) mounted within the scope 38. The illumination fibers 42 are embedded within an epoxy material 44, and terminate at a viewing or distal end 46 of the scope 38 of the arthroscope scope assembly 32 for providing illumination for viewing the interior of a joint such as the knee 14 (Figure 1). The illumination fibers 42 are joined in a fiber bundle 48 (Figure 2).

A front tube 52 is mounted within the housing 30. The front tube 52 is generally cylindrical in shape and is removably attached to the housing 30. The light source 35 is mounted within the front tube 52. The front tube 52 also houses a sapphire window 54 in light communication with an image guide 58 of the scope 38. The sapphire window 54 in turn, is in light communication with focusing optics 56 mounted within the front tube 52.

The focusing optics 56 are mounted in an optics base 60 slidingly mounted in the front tube 52. Thus, an image from the image guide 58 which has passed through the sapphire window 54 can enter the focusing optics 56. As previously stated the image guide 58 functions to transfer an image from the plane of a GRIN lens attached to the distal or viewing end of the image guide 58 to the focusing optics 56. Preferably, the focusing optics 56 magnify light from the sapphire window 54 several times (i.e. 7X) and focus this light onto a camera head 62 which is also mounted within the housing 30.. In a preferred embodiment the camera head 62 is a charged coupled device (CCD).

Referring now to Figure 2A the front tube 52, light source 35, and light transmitting means 39 are shown in schematic form. The internal light source 35 includes the micro light 36 mounted along a center line 72. The internal light source 35 also includes the light transmitting means 39 for transmitting light from the micro light 36 to the illumination fibers 48 of the illumination fiber bundle 48 of the scope 38.

The light transmitting means 39 may include a fiber optic taper 80 that funnels light from the micro light 36 into the illumination fiber bundle 48. The light transmitting means may also include a parabolic reflecting mirror 84 for reflecting some of the light output from the micro light 36 onto the illumination fiber bundle. In addition, the light transmitting means 39 may include an illumination GRIN lens 50 juxtaposed to a proximal end of the illumination fiber bundle 42 for collecting and channeling light into the fiber bundle 42. With a disposable scope 38 such a GRIN lens 50 would be integrated into illumination optics located within the housing 30. Finally the light transmitting means may include an integrating sphere 86. Such an integrating sphere 86 may be in the form of a coated spherical bulb that functions to collect all of the light from an internal light source and allows the light to escape through a single opening.

The micro light bulb 36 is preferably a miniature light bulb of the type that produces a relatively high output for its size. By way of example and not limitation, a 5VDC, 1.4 A, Halogen technical lamp such as a focused lens end lamp, that produces approximately 20,000 LUX output is suitable for this application. An external power line 112 (Figure 2) may be provided for the micro light 36. With such a micro light 36 as a light source it is essential that most of the output of the micro light 36 be transmitted to the illumination fiber bundle 48.

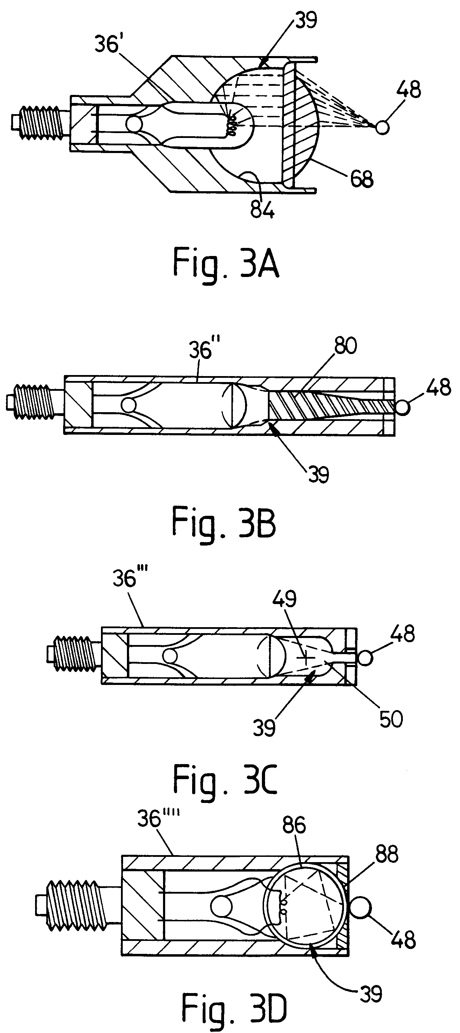

Figure 3A-3D illustrate various constructions for the micro light 36 and light transmitting means 39. With reference to Figure 3A, a parabolic reflecting mirror 84 is positioned around a micro light 36' to reflect light from the micro light 36'. The reflecting mirror 84 is shaped as a paraboloid of revolution. The parabolic reflecting mirror 84 forms the reflected light into a column of light which is passed through a focusing lens 68 and focused on the illumination fiber bundle 48. The focusing lens 68 may be in the form of an aspherical lens.

With reference to Figure 3B a micro light 36'' may be in light communication with a taper section 80 which is adapted to funnel light from the micro light 36'' into the illumination fiber bundle 48. The taper section 80 may be formed of a fiber optic material having and an antireflection (AR) coating. Such an AR coating may be selected to reduce reflection losses (i.e Fresnel) occurring at the surface of the taper section 80. Moreover the taper section 80 may be formed with a taper angle which closely matches the numerical aperture (N.A.) of the illumination fiber bundle 48 (or a GRIN lens rod 50 attached to the illumination fiber bundle 48). This taper angle enables an illumination output cone from the taper section 80 to be directed at the illuminating fiber bundle 48 at a known angle of illumination. As an example such a micro light 36'' may be a commercially available bulb having an integral focusing lens.

With reference to Figure 3C, a focusing lens light bulb 36''' may be adapted to focus light trough a focal plane 49 and a GRIN rod 50 and onto the illumination fiber bundle 48. Such a focusing lens light bulb 36''' may also be a bulb with an integral focusing lens.

With reference to Figure 3D a light bulb 36'''' may include an integrating sphere 86 coated inside with a highly reflective white coating. A small uncoated area 88 on the spherical bulb allows light to escape only at selected angles. These angles are chosen to be within the N.A. of the illuminating fibers 42 of the illumination fiber bundle 48.

With any of the arrangements shown in Figures 3A-3D, essentially all of the light output from the micro light 36 is transmitted to the illumination fiber bundle 48. The objective is to collect the maximum light energy and transfer this light to the illumination fiber bundle 48. This light then travels through the individual illumination fibers 42 of the fiber bundle 48 and out the viewing end 46 of the flexible scope 40 to illuminate the internal structure of the body. This light is then collected by the image guide 58 of the scope 38 and transmitted through the sapphire window 54 and focusing optics 56 to form an image of the body.

In addition to providing the above structures for a micro light 36 and light transmitting means 39 shown in Figure 3A-3D for transmitting light from the micro light 36 to the illumination fiber bundle 48, it is also advantageous to overdrive the micro light 36 into the color spectrum best suited for the arthroscope 10. In general such a shift is into the blue visible light area and away from the infra-red light area. This shift also increases the total photon flux.

Using a micro light 36 and light transmitting means 39 (Figure 3A-3D) constructed in accordance with the invention the final light output at the viewing end 46 of the flexible scope assembly 32 is equivalent to that of an external light source which uses a high powered Xenon or Metal Halide light bulb. With such external systems it is not unusual to lose up to 98% of the original light energy by the time it has been transmitted to the point of application.

To enable the use of a micro light 36 in an endoscope system such as arthroscope 10 having a relatively small illuminating bundle 48 and to produce a high quality image, it is also necessary that the light collecting system (i.e., image guide 58) and imaging system relating to the camera assembly 62 (CCD) be optimized. Some system parameters which can be controlled to achieve these results include the following.
1. Utilizing an image collecting lens such as a GRIN lens that has a matching index with the image guide 58 (Figure 2) at their common interface.
2. Coating the sapphire lens 54 (Figure 2) with an anti-reflection material (AR) to reduce surface reflection losses.
3. The focusing optics 56 (Figure 2) or relay lens system, either fixed or zoom design, must be optically designed to suit all the input and output parameters of the imaging system.
4. To balance the illumination system micro computer electronic shuttering of the CCD 62 (Figure 2) video output must be accomplished. This enables the optical system to be more sensitive to changes in illumination on the CCD 62 surface, and yields an improved image onto a CRT 26 for recording onto the VCR 28 (Figure 1).
5. White balance selection, either automatic or manual, can be used for electronic comparison of this color to set the spectral color content (or spectral balance) of the picture. This feature is essential if a micro light 36 not having the ideal light spectrum in the visual range, is to be made acceptable.
6. The image level signal/noise ratio must be optimized for producing a high quality video image.

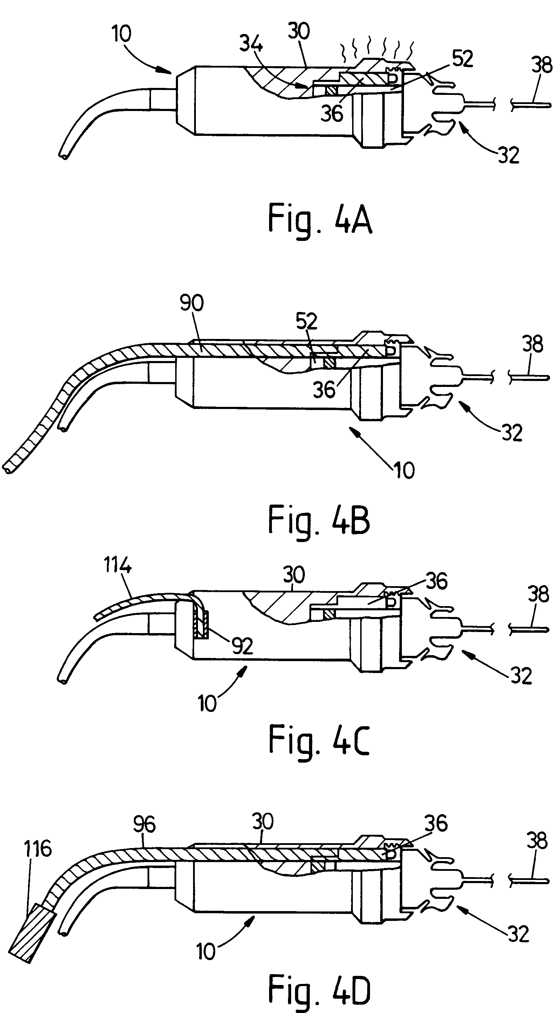

In addition to the above-described structure and operational parameter for a light source 35 and light transmitting means 39, an essential part of the present invention is to be able to dissipate heat generated by the micro light 36 in its confined space within the housing 30. This can be done passively by conducting the heat away or by cooling with a coolant. Four methods are illustrated in Figures 4A-4D for dissipating heat.
1. As shown in Figure 4A, heat can be dissipated by using conventional methods of convecting the heat away. These conventional methods include:
   A. Conducting heat from the micro light 36 to the housing 30 where it is convected away using a conductive medium such as thermal grease.
   B. Selectively shaping the housing 30 (i.e. added thickness) to spread heat more evenly and to conduct the heat away.
   C. Ribbing the housing 30 for dissipating and radiating the heat away.
   D. Coating the inside of the housing 30 with a black material for heat absorption.
   E. Plating the outside of the front tube 52 with a black surface for radiation of heat.
   F. Plating the outside of the camera assembly 34 bright aluminum for reflection of heat from the camera assembly 34.
2. As shown in Figure 4B heat can also be dissipated by using a chemical transfer method such as a hollow tube or a heat pipe 90 filled with a low vapor pressure, low boiling point liquid that is adapted to carry heat away from the housing.
3. As shown in Figure 4C heat can also be dissipated by installing a Peltier semiconductor heat pump chip 92 and heat pipe 114.
4. As shown in Figure 4D heat can be dissipated by using a separate lamp holder 116 that clips to external cables 96 connected to the housing 30.

### ALTERNATE EMBODIMENT

Referring now to Figures 5A-5C several alternate embodiment endoscope assemblies or arthroscopes constructed in accordance with the present invention are shown.

In general to make a cableless, totally self contained endoscope, a battery pack may be utilized together with a microscopic eyepiece.

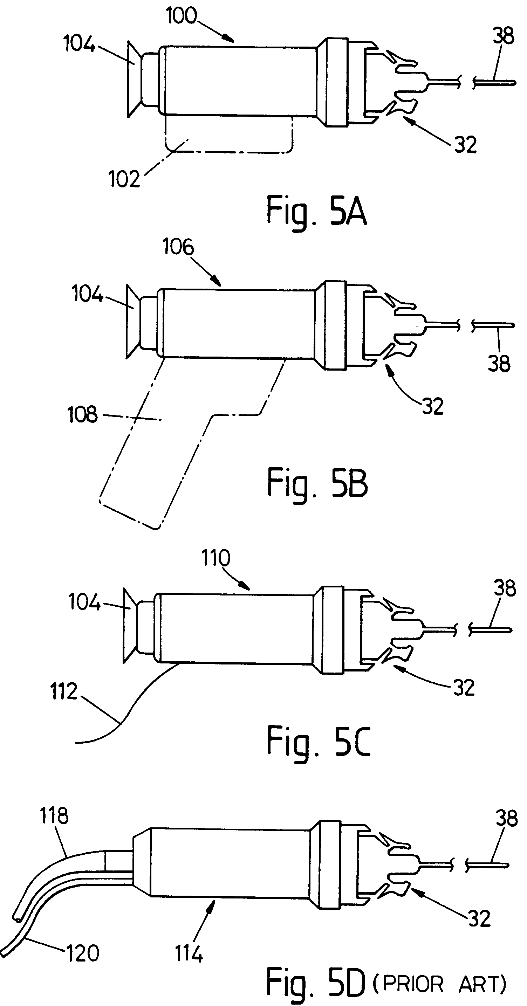

With reference to Figure 5A, an arthroscope 100 includes battery pack 102 as an internal power source and an eyepiece 104. Such an arthroscope 100 may also include an internal light source 36 as previously explained. With reference to Figure 5B arthroscope 106 may include an eyepiece 104 and a battery pack 108 formed as a handle.

With reference to Figure 5C an arthroscope 110 may include an eyepiece 104 and a power line 112 to an external power source. As comparison and with reference to Figure 5D the cable assembly of a prior art arthroscope 114 typically includes;
a. camera control unit cable 118 to the camera assembly;
b. a heavy fiber optics illumination cable 120.

With an arthroscope constructed in accordance with the invention the heavy and expensive fiber optic cable 118 is eliminated as the internal light source 35 provides the light. With an externally powered internal light source 35 this fiber optic cable 120 can be replaced by a small power cable 112 (Figure 5C). With the embodiments having an internal power source (100-Fig 5A, 106-Fig 5B) the power cable is also eliminated

Thus an endoscope can be constructed in accordance with the invention with an internal light source. Such and endoscope may be constructed and produce an image that is equivalent to images produced with endoscopes having large external light sources.

While the particular endoscope as herein shown and disclosed in detail is fully capable of obtaining the objects and providing the advantages herein before stated, it is to be understood that they are merely illustrative of the presently preferred embodiments of the invention and that no limitations are intended to the details of construction or design herein shown other than as described in the appended claims.

## Claims

1. An endoscope assembly for examining an internal structure of a body, characterised in that it comprises:
a housing;
a disposable scope assembly including a scope detachably connected to the housing and including an optical illumination fiber for illuminating the internal structure of the body;
an internal light source mounted within the housing in light communication with the optical illumination fiber;
means for transmitting light from the internal light source to the optical illumination fiber; and
means in light communication with the scope assembly for generating a visual display image representative of the illuminated structure of the body.

2. An endoscope according to claim 1 wherein;
the scope has a proximal end and a distal end insertable into a body for examining the internal structure thereof, and the scope includes an optical fiber image guide having a distal end for gathering light from the internal structure and guiding the light through the scope, and the scope further includes a plurality of optical illumination fibers positioned next to the image guide for illuminating the structure;
the housing is connected to the proximal end of the scope for supporting the scope and the housing includes an image generating means in light communication with the image guide for generating an image of the internal structure;
the internal light source includes a miniature light bulb connected to a power source; and
wherein the endoscope further comprises heat dissipation means for dissipating heat generated by the light bulb from the housing.

3. An endoscope according to claim 1 or 2 wherein the light transmitting means includes an integrating sphere for directing light from the light source at the optical illumination fiber.

4. An endoscope according to any one of claims 1 to 3 wherein the light transmitting means includes an optical taper section for transmitting light from the light source into the optical illumination fiber at an angle which matches a numerical aperture (N.A.) of the optical illumination fiber.

5. An endoscope as recited in claim 2 and wherein the light transmitting means includes a GRIN lens having a matching N.A. with the optical illumination fiber and adapted to transfer light from the light source onto the optical illumination fiber.

6. An endoscope according to claim 5, wherein the GRIN lens is mounted to an illumination system for the optical illumination fibers juxtaposed with a proximal end of the optical illumination fiber for directing light from the light bulb into the optical illumination fiber.

7. An endoscope according to any one of the preceding claims wherein the light transmitting means includes a parabolic reflecting mirror with a focusing lens mounted around the light bulb and adapted to receive light from the light bulb and reflect the light in a column onto the optical illumination fiber.

8. An endoscope according to any one of the preceding claims further comprising a conduit for providing power to the light source from an external source.

9. An endoscope according to any one of the preceding claims further comprising a battery mounted to the housing for powering the light source.

10. An endoscope according to any one of the preceding claims further comprising a microscopic lens mounted to the housing and adapted to view images from the scope assembly.

11. An endoscope according to any one of the preceding claims which is constructed as an arthroscope.

12. An endoscope according to any one of claims 2 to 11 wherein the heat dissipation means includes means for providing heat conduction to and heat convection away from the housing.

13. An endoscope according to any one of claims 2 to 11 wherein the heat dissipation means includes a heat pipe attached to the housing and filled with a liquid for transferring heat generated by the light source out of the housing.

14. An endoscope according to claims 2 to 11 wherein the heat dissipation means includes a peltier heat pump and heat pipe attached to the housing.

15. An endoscope according to any one of claims 2 to 11 wherein the heat dissipation means includes mounting the miniature light bulb on a separate holder attached to the housing.

16. An endoscope according to any one of the preceding claims which is hand-held and portable and wherein the housing includes a camera head in light communication with the image guide for generating a signal representative of the light from the internal structure.

17. An endoscope according to claim 16 and further comprising a lens coupled to the camera head for viewing an image produced by the scope assembly.

18. An endoscope according to claim 16 or 17 wherein the light bulb is overdriven by the power source.

19. An endoscope according to any one of claims 16 to 18 and wherein the light bulb includes an integral focusing lens adapted to focus light on a fiber optic taper having a taper angle that matches a N.A. of the illumination fibers.

20. An endoscope according to any one of claims 16 to 18 and wherein the light bulb includes an spherical bulb coated with a highly reflective coating and having an uncoated area adapted to allow light to escape at an angle that matches a N.A. of the illumination fibers.
